(19) Europäisches Patentamt
European Patent Office
Office européen des brevets

(11) **EP 0 993 310 B1**

(12) **EUROPEAN PATENT SPECIFICATION**

(45) Date of publication and mention
of the grant of the patent:
**14.01.2009 Bulletin 2009/03**

(51) Int Cl.:
*A61L 15/24* (2006.01)   *A61L 15/42* (2006.01)
*A61L 15/60* (2006.01)

(21) Application number: **98929984.7**

(22) Date of filing: **12.06.1998**

(86) International application number:
**PCT/SE1998/001129**

(87) International publication number:
**WO 1998/057675 (23.12.1998 Gazette 1998/51)**

(54) **A METHOD FOR THE MANUFACTURE OF AN ABSORBENT MATERIAL STRUCTURE**

EIN VERFAHREN ZUR HERSTELLUNG EINER STRUKTUR AUS ABSORBIERENDEM MATERIAL

PROCÉDÉ DE FABRICATION DE STRUCTURE DE MATIERE ABSORBANTE

(84) Designated Contracting States:
**AT BE CH CY DE DK ES FI FR GB GR IE IT LI LU MC NL PT SE**

(30) Priority: **19.06.1997 SE 9702350**

(43) Date of publication of application:
**19.04.2000 Bulletin 2000/16**

(73) Proprietor: **SCA Hygiene Products AB**
**405 03 Göteborg (SE)**

(72) Inventors:
• **KALENTUN, Pia**
**S-413 14 Göteborg (SE)**
• **SCHMID, Andrea**
**S-435 42 Mölnlycke (SE)**
• **BUSCHKA, Anette**
**S-416 79 Göteborg (SE)**
• **LUNDSTRÖM, Kerstin**
**S-412 64 Göteborg (SE)**

(74) Representative: **Romare, Laila Anette et al**
**Albihns AB**
**P.O. Box 142**
**401 22 Göteborg (SE)**

(56) References cited:
**EP-A- 0 156 541      WO-A-93/04093**
**WO-A-94/22502**

**Description**

TECHNICAL FIELD:

The present invention pertains to a manufacturing method for an absorbent material structure for absorbing liquids.

The absorbent material structure produced according to the invention has the form of an open-celled, flexible foam and provides highly absorbent properties with the aid of ionic groups which have been created in the material structure by means of hydrolysis. The material structure produced according to the invention is primarily intended for use in absorbent disposable articles such as baby diapers, incontinence protectors and products for feminine hygiene, but is also adapted for use in other absorbent articles, such as for example in different wiping materials, bandage materials and other similar products.

BACKGROUND OF THE INVENTION:

The development of absorbent disposable articles, such as baby diapers, incontinence protectors and products for feminine hygiene, has recently to a high degree been directed towards the development of thinner and thinner products. The reason why the product development has taken this direction is, for example, that a thinner diaper is more comfortable to wear, and that it is perceived as being more discreet by the user. Furthermore, a thinner product results in a lower distribution cost and a lower environmental load per article in connection with the transports which are required in order to enable the product to reach the enduser.

The possibilities to provide thinner and thinner absorbent articles are dependent on the ability to develope thinner and thinner absorption cores or absorbent structures, which can receive and store large quantities of human exudates, such as urine or other body fluids.

Thereby, particle-shaped absorbent polymers, often referred to as superabsorbents, have been particularly valuable in order to enable this development. A commonly occurring type of superabsorbents is polyacrylates with a relatively low degree of cross-linking.

The absorption mechanism of superabsorbents of polyacrylate type is based on the fact that the lightly cross-linked polymers comprise a plurality of anionic carboxylate groups which are attached to the polymer chain. These charged carboxylate groups enable the polymer to absorb aqueous liquids by means of osmotic forces.

Another important mechanism, where absorbent articles are concerned, is absorption based on capillary forces. Examples of capillary absorbents are different fibre-based materials such as fluff pulp, tissue paper or different types of nonwoven materials. Under certain circumstances, capillary absorbents can be superior to the above-mentioned absorbents acting through osmotic forces. Accordingly, the absorption and distribution rates are in general higher for capillary absorbents than for superabsorbents. By means of placing capillary absorbents having a certain fibre orientation, capillary size or hydrophilic character in suitable positions in an absorbent article, the absorbed liquid may further be directed in the desired direction.

Therefore, many previously known absorbent articles combine capillary absorbents with superabsorbents, acting by means of osmotic forces, with good results.

In the following, such an advantageous previously known combination is exemplified by an imagined absorbent body intended for use in a baby diaper.

The imagined absorbent body is covered with a hydrophobic surface material, having relatively large capillaries, on the side of the absorbent body which is intended to be facing towards a wearer. This surface layer has the ability to rapidly let through, for example urine, to underlying material layers, and remains dry due to its hydrophobic nature, something which is an advantage, amongst other things where comfort is concerned.

An acquisition layer, e.g. a cellulose wadding, is arranged inside the surface material of the imaginary absorbent body. The function of the acquisition layer is to let liquid through to the absorbent core.

An absorbent core, consisting of a mixture of fluff pulp fibres and superabsorbent, follows inside the acquisition layer of the imaginary absorbent body. Inside the core, the pulp fibres can be said to form a fibre matrix which distributes the liquid in the core by means of capillary forces, so that the superabsorbent particles gradually can absorb and store the liquid by means of osmotic forces.

Absorbent articles of the "combination type" described in the example above, however, are relatively expensive and complicated to manufacture, since they contain a number of different material types. Another disadvantage is that problems with the integrity of the absorbent core easily may arise during use, so that the core gradually ruptures or becomes lumpy. Another difficulty is to be able to distribute and retain the superabsorbent in a desired way in the absorbent core until the absorbent article has been used.

Another problem which may arise with combination products comprising both fluff pulp and particle-shaped superabsorbents is so-called gel-blocking. This problem arises because superabsorbent particles, which have absorbed liquid, locally form a gel. This results in the capillary liquid transport via the fibre matrix being blocked and therefore causes a collection of liquid in certain portions of the absorbent body, whereas the absorption capacity in other portions remains more or less unemployed.

In light of the above-mentioned problems, it has previously been suggested that another type of absorbent material, namely polymeric foams with open cells, should be used in absorbent articles.

Such previously known foam materials can be manufactured using the manufacturing method which is disclosed in the patent publication WO 93/21234. The therein disclosed manufacturing method provides porous, cross-linked polymeric foam materials having a low density.

According to WO 93/21234, the manufacturing method comprises a "water-in-oil" emulsion polymerization of monomers which comprise at least one vinyl monomer, a difunctional unsaturated cross-linking monomer, at least 90 weight-% water calculated on the emulsion, a first surfactant comprising a sorbitan monoester having a fatty acid moiety of at least 6 carbon atoms, and at least one second sorbitan ester having at least one different fatty acid moiety than the first surfactant, and a polymerization catalyst. The method is claimed to provide open-cellular foams with low density, high absorption capacity and good physical properties in this way.

In the above-mentioned WO 93/21234, it is disclosed that the internal water phase during polymerization preferably contains a water soluble electrolyte, amongst other things in order to make the foam more water wettable. Suitable electrolytes for this purpose are stated to be inorganic salts (monovalent, divalent, trivalent or mixtures thereof), such as alkali metal salts, alkaline metal salts, and heavy metal salts such as halides, sulphates, carbonates, phosphates and mixtures thereof. The electrolytes can also include sodium chloride, sodium sulphate, potassium chloride, potassium sulphate, lithium chloride, magnesium chloride, calcium chloride, magnesium sulphate, aluminium chloride and mixtures of these. Mono-or di-valent metal salts with monovalent anions are stated to be preferred.

Foam materials of the type which is disclosed in WO 93/21234 can provide both capillary liquid absorption, transportation and storage and should therefore be suitable for use as absorption cores in absorbent articles, for example diapers.

Accordingly, the patent publication WO 93/04092 discloses absorbent foam materials for absorption of aqueous body fluids. The foam materials disclosed in WO 93/04092 are said to be suitable for use in absorption cores of absorbent articles, for example diapers.

The foam materials according to WO 93/04092 comprise hydrophilic, flexible structures with open cells which preferably are prepared by means of polymerizing "water-in-oil" emulsions. Such emulsions, having a high proportion of water phase, i.e. more than 70 volume-% "internal phase" and a low proportion of oil phase, are referred to as HIPE-emulsions (High Internal Phase Emulsions).

According to WO 93/04092, HIPE-emulsions can be formed by means of intense agitation of a polymerizable mixture containing a relatively small amount of a polymerizable monomer-containing oil phase and a, relatively, larger amount of a relatively monomer-free water phase. In the emulsion, this discontinuous, "internal" water phase forms a network of dispersed "droplets", which are surrounded by the continuous, polymerizable oil phase with therein dissolved surfactant. During the polymerization of the monomers in the continuous oil phase, a cellular foam structure is formed. After polymerization, the aqueous liquid remaining in the foam structure formed upon polymerization can be removed by pressing and/or drying the foam. If necessary, the polymerized foam material can be subjected to different types of post-treatment, for example a hydrophilizing treatment.

(Comment: In connection with this, it can be mentioned that the inventors of the present invention have found that HIPE-emulsions also can be formed without any intense agitation.)

In WO 93/04092, a number of different parameters are mentioned which are essential for deciding structural, mechanical and functional parameters of a foam material.

Accordingly, it is disclosed that the relative amounts of water and oil phase which are used in order to form the polymeric foam can affect the foam density, the cell size and the specific surface of the foam, and the dimensions of the cavities which form the foam. In WO 93/04092 it is disclosed that the ratio between water and oil phase suitably is between 12:1 and 100:1, more preferably between 20:1 and 70:1, and most preferably 25:1 to 50:1.

According to WO 93/04092, the continuous oil phase of the emulsions comprises the polymers which are to form the solid foam structure. The monomers are said to include three different components; principal monomer, comonomer and cross-linking agent. The principal monomer is said to comprise one or more monomers that tend to impart glass-like properties to the resulting foam structure. After polymerization, such monomer materials are said to produce homopolymers with high molecular weight (> 6000) and a glass transition temperature $T_g$ about 40 °C or higher. As preferred "glass-like" monomers, inter alia, non-substituted or substituted styrenes are mentioned. The addition is said to normally be between 3-41%, more preferably 7-40 weight-% of the oil phase.

The above-mentioned comonomer component is claimed to comprise one or several comonomers, which tend to impart "rubber-like" properties to the resulting foam structure. This type of comonomers is said to be monomers which would provide homopolymers with a high molecular weight (> 10000) and a glass transition temperature of about 40 °C or lower. Monofunctional, "rubber-like" comonomers of this type are said to comprise, for example, alkyl-acrylates, alkyl-methacrylates, allyl-acrylate, butadiene, substituted butadienes, vinylidene halides and combinations of such. Preferred "rubber-like" comonomers are said to include; butylacrylate, 2-ethylhexylacrylate, butadiene, isoprene and combinations of these. Butylacrylate and 2-ethylhexylacrylate are stated to be the most preferred. The monofunctional "rubber-like" comonomer component is said to normally constitute up to 27-73 %, more preferably from about 27-66 weight-% of the oil phase.

(In connection with this, it might be mentioned that it is generally accepted that glass transition temperatures $T_g < 25$ °C result in rubber-like properties at room temperature, for example, polymers comprising 2-ethylhexylacrylate have $T_g$ = -70 °C and are therefore clearly rubber-like at room temperature).

In WO 93/04092, it is disclosed that the molar ratio between the "glass-like" principal monomer component and the "rubber-like" comonomer in general is between 1:25 to 1.5:1, more preferably from about 1:9 to 1.5:1.

In order to make it possible to obtain a polymerization, the HIPE-emulsions disclosed in WO 93/04092 are said to also require the presence of a poly-functional cross-linking agent in the oil phase. Thereby, the selection of cross-linking agent is said to be of great importance for achieving the desired properties of the finished foam. It is disclosed that the cross-linking agent can be selected from a great number of polyfunctional, preferably difunctional monomers, for example aromatic divinyls such as divinylbenzene, divinyltoluene or diallylphthalate. Alternatively, aliphatic cross-linking agents of divinyl type are said to be useful, such as diacrylic acid esters of polyols. However, the most preferred cross-linking agent is claimed to be divinylbenzene. Suitable addition levels are said to be 8-40 %, more preferably 10 to 25 weight-% of the oil phase.

According to WO 93/04092, it is of uttermost importance that the above-mentioned monomers, comonomers and cross-linking agents are substantially water-insoluble, so that they primarily are soluble in the oil phase and not in the water phase. Furthermore, the importance that the raw materials used for the foam preparation are non-toxic and chemically stable is underlined.

According to WO 93/04092, another essential component of the oil phase of HIPE-emulsions is an emulsifier, which allows the formation of a stable HIPE-emulsion. Such emulsifiers are soluble in the oil phase, used when forming the emulsion, and are said to be nonionic, cationic or anionic, as long as they are able to form a stable emulsion. Preferred emulsifiers are said to be sorbitan fatty acid ethers, polyglycerol fatty acid esters, polyoxyethylene (POE), fatty acids and esters. Sorbitan monolaurate, sorbitan monooleate and combinations of sorbitan trioleate and sorbitan monooleate are mentioned as particularly preferred. Other preferred emulsifiers are said to be polyglycerol ester and sorbitan sesquioleate. The emulsifier is said to normally constitute about 2-33 weight-%, more preferably 4-25 weight-% of the oil phase.

In WO 93/04092, it is disclosed that the oil phase, in addition to the above-mentioned monomers and emulsifiers, also may contain further optional components. Examples of such are said to be oil soluble polymerization initiators or solvents for solving monomers and emulsifiers in the oil phase.

According to WO 93/04092, the discontinuous, internal water phase in a HIPE-emulsion is an aqueous solution, which in general contains one or more dissolved components. An essential such component is said to be a water soluble electrolyte, the task of which is to minimize the tendency of monomers and cross-linking agents, which primarily are oil soluble, to also dissolve in the water phase. This is said to be important in order to minimize the risk of polymeric material filling the cells which are formed by the water phase droplets in the emulsion, something which could result in the final foam quality being impaired.

The above-mentioned electrolyte can allegedly be of any type that provides ionic strength to the water phase. Preferred electrolytes are said to be mono-, di-, or trivalent inorganic salts, such as water soluble halides, e.g. chlorides, nitrates and sulphates of alkali metals or alkaline earth metals. Sodium chloride, calcium chloride, sodium sulphate and magnesium sulphate are mentioned as examples, with calcium chloride as the most preferred. The electrolyte addition is said to be between 0.2-40% in general, more preferably from about 0.5-20 weight-% of the water phase.

From WO 93/04092, it is further evident that a polymerization initiator usually is added to the water phase. Such an initiator is said to be any conventional water-soluble initiator of free radicals. The initiator is said to consist of compounds such as sodium-, ammonium- and ammoniumpersulphates, caprylyl peroxide, benzoyl peroxide, hydrogen peroxide, cumene hydroperoxides, tertiary butyldiperphtalate, tertiary butylperbenzoate, sodium peracetate, sodium percarbonate and the like. Also conventional redox initiator systems are said to be useful, by means of combining the above-mentioned peroxygen compounds with reducing agents, such as sodium bisulphite, L-ascorbic acid or ferrous salts. The initiator is said to constitute up to about 5 mole-%, more preferably 0.001-0.5 mole-%, based on the total number of moles of polymerizable monomers in the oil phase. Where addition to the water phase is concerned, this is said to involve contents of 0.02-0.4 %, more advantageously from about 0.1-0.2 weight-% of the water phase.

WO 93/04092 discloses that the above-mentioned oil and water phases are combined during agitation in order to form a stable emulsion, after which the emulsion is subjected to polymerization conditions which are suitable to bring about polymerization of the monomers in the oil phase and thereby to form a solid cellular foam structure.

From WO 93/04092, it is evident that the therein disclosed foam material has a hydrophobic character immediately after the polymerization. Since the intended use requires a hydrophilic material, the foam material has to be treated after the polymerization in order to render the surfaces of the material more hydrophilic. This so-called hydrophilizing treatment is said to be accomplished by means of treating the foam structure with a suitable hydrophilizing agent.

According to WO 93/04092, the hydrophilizing agent may be any material, having the ability to enhance the wettability by water of the polymer surfaces. Such previously known agents are said to include surface-active materials, surfactants, of anionic, cationic or nonionic type. Hydrophilizing agents are said to normally be employed in liquid form, thereby dissolved in water in order to form an aqueous hydrophilizing solution which is applied onto the surfaces of the foam material. It is claimed that a sufficient amount of hydrophilizing agent can be absorbed in this way, in order to render the foam surfaces sufficiently hydrophilic, without affecting the flexibility and compressibility properties of the foam material. The content of hydrophilizing agent, which has been incorporated into the foam structure, is said to suitably be between 0.1-10 weight-% of the foam.

Suitable hydrophilizing agents are claimed to comprise mild, non-irritating surfactants which are applied on the foam structure in amounts which are sufficient in order to result in a surfactant content from about 0.5-5.0%, more preferably from about 1-3 weight-% of the foam.

As examples of suitable surfactants, WO 93/04092 mentions alkyl sulphates and alkylethoxylated sulphates of the type which is used in commercial washing-up detergents. Aqueous solutions of such surfactants are said to typically be used for washing the foam structure, either residual water material after the foam polymerization, or more preferably as part of a washing treatment that serves to remove this residual water phase material.

Another preferred type of hydrophilizing agents is said to be hydratable, and preferably hygroscopic or deliquescent, water soluble inorganic salts, such as alkaline earth metal salts. Preferred salt types are claimed to be calcium and magnesium halides, such as calcium chloride. It is stated that salts of this type can be easily incorporated in the absorbent foam materials by means of treating the foam materials with solutions of such salts. In the same way as mentioned above with regard to surfactants, it is disclosed that the hydrophilizing treatment either can be performed after water phase residues from the polymerization have been removed, or as a part of the process of removing the residual water phase from the recently polymerized foam. Preferred salt contents are claimed to be from about 0.1-7 weight-% of the foam material.

From WO 93/04092, it is evident that the hydrophilizing treatment is performed to the extent it is necessary on HIPE-foam materials, which after polymerization are comparatively hydrophobic in nature.

In WO 93/04092, it is also disclosed that some HIPE-foam materials can be sufficiently hydrophilic directly after the polymerization, and that such materials therefore do not need any subsequent hydrophilizing treatment. As examples of such materials, HIPE-foams wherein sorbitan fatty acid esters have been used as an emulsifier additive in the oil phase, and wherein calcium chloride has been used as an electrolyte in the water phase, are mentioned. It is claimed that the content of calcium chloride in the residual water phase of such materials, after the polymerization, is sufficiently high in order to render the internal surfaces of the foam material, which also contain emulsifier residues, suitably hydrophilic also after the polymerized foam has been dewatered.

There are a further number of patent publications which disclose the preparation of hydrophilic foam materials of the above-discussed type, or materials of a similar type.

Among these, the patent publication WO 93/04093 can be mentioned, disclosing a process for continuous preparation of HIPE-emulsions which are suitable for subsequent polymerization into polymeric foam materials, that upon dewatering act as absorbents for aqueous body fluids. The process involves continuous addition of a certain type of monomer-containing oil phase and a certain type of electrolyte-containing water phase to a dynamic mixing zone at relatively low water:oil ratios. The flow rates are steadily adjusted in order to increase the water:oil ratio of the streams which are fed to the continuous mixing zone, while the content in the dynamic mixing zone is subjected to a shear agitation which is sufficient in order to form a HIPE-emulsion, which upon subsequent polymerization provides a foam which has an average cell size from about 5 to 100 micrometer. The formation of such a stable HIPE-emulsion is completed by feeding the contents in the dynamic mixing zone to and through a static mixing zone.

In WO 93/04093, a number of different emulsifiers are mentioned as being useful for creating a stable HIPE-emulsion. The emulsifiers are claimed to be nonionic, cationic, anionic or amphoteric, depending on the other conditions. Sorbitan fatty acid esters, polyglycerol fatty acid esters, polyoxy-ethylene fatty esters and esters are mentioned as particularly preferred emulsifiers.

In WO 93/04093, it is further disclosed that the foam materials prepared by means of the therein described process, generally, should be subsequently treated in order to render the foam materials suitable as liquid absorbents. It is claimed that such a subsequent treatment can comprise a washing, in order to remove residual water phase from the cells of the foam, followed by a hydrophilizing treatment in order to render the surfaces of the foam more hydrophilic. Thereby, calcium chloride is mentioned as a suitable hydrophilizing agent.

WO 93/04113 is another patent publication within the field of hydrophilic foam materials. In this publication, a method for rendering hydrophobic foams hydrophilic by means of treatment with simple surfactants and salts as hydrophilizing agents is disclosed. In the disclosed method, a surfactant-containing foam is treated with a solution of, for example, calcium chloride and is thereafter dried in order to leave a uniformly distributed residue of hydrated or hydratable calcium chloride on the surfactant-containing internal foam surfaces. Also other hydratable calcium or magnesium salts, such as magnesium chloride, are claimed to be usable. The resulting hydrophilic foams are said to be suitable for use in absorbent devices, including diapers, sanitary napkins, bandages and the like.

Another patent publication within the discussed field is WO 93/04115. This discloses a method for hydrophilizing hydrophobic foams, such as polyurethane foams and polymerized "water-in-oil" emulsion foams, which are claimed to be possible to hydrophilize with sorbitan monolaurate, which after drying is said to leave an essentially uniform residue of sorbitanmonolaurate on the internal foam surfaces. The resulting treated foams are said to become hydrophilic and, accordingly, become suitable for use in absorbent devices, including diapers, sanitary napkins, bandages and the like.

A further patent publication, WO 94/13704, relates to an absorbent foam material for aqueous body fluids and a manufacturing process for the same. The patent publication discloses a relatively thin, collapsed / non-expanded polymeric foam material which, when in contact with aqueous body fluids, expands and absorbs the fluids. Furthermore, a process for obtaining such foam materials in a stable way is disclosed, by means of polymerizing a specific type of "water-in-oil" emulsion, normally known as a HIPE-emulsion.

The foam materials disclosed in WO 94/13704 are said to be hydrophilic in character. It is claimed that hydrophilic foam surfaces are obtained, due to residues of hydrophilizing agent having remained in the foam structure after polymerization, or by means of a special subsequent treatment procedure, aimed at modifying the surface energy of the foam material.

An example of remaining residues of hydrophilizing agent in the foam structure, which is disclosed in WO 94/13704, is a toxilogically acceptable, hygroscopic, hydrated salt, which preferably is calcium chloride.

According to WO 94/13704, further examples of such residues of hydrophilizing agents are certain oil-soluble emulsifiers, for example sorbitanlaurate.

In WO 94/13704, it is disclosed that both types of hydrophilizing agents either can be added as raw material for the foaming process, or in a subsequent treatment step.

The patent publication US 5 500 451 discloses a process for preparing HIPE-emulsions, which can be polymerized in order to provide flexible, micro-porous, open-celled polymeric foam materials. The prepared foam materials are claimed to be able to absorb aqueous liquids such as urine. It is disclosed that HIPE-emulsions can be manufactured by means of using certain aliphatic polyglycerol ethers as emulsifiers. It is claimed that these are chemically less complex and vary less in composition than many previously utilized emulsifiers for HIPE-emulsions. Furthermore, the polyglycerol ethers are claimed to have higher contents of surface-active components and lower contents of undesired components than previously utilized emulsifiers. It is claimed that the polyglycerol ethers provide emulsions with water droplets of a comparatively uniform size which are dispersed in the continuous oil phase.

The aliphatic polyglycerol ethers disclosed in US 5 500 451, which are used as emulsifiers, are said to be selected from the group consisting of aliphatic glycerol ethers comprising at least about 40% linear, mono-aliphatic diglycerol ethers and at least about 60% aliphatic polyglycerol ethers which have aliphatic groups within the region $C_{10}$-$C_{24}$. By means of utilizing aliphatic polyglycerol ethers with aliphatic groups within the region $C_{12}$-$C_{18}$, it is claimed that a hydrophilizing effect is maintained in the finished HIPE-foam also after washing and dewatering. EP-A-0 156 541 is directed to a rubber-like polymeric foam comprising ionic groups formed through acidic hydrolysis.

Accordingly, flexible, open-celled foam materials which are hydrophilic directly after the polymerization are previously known, as well as foam materials which have been rendered hydrophilic by means of subsequent treatment with different surfactants or electrolytes. The use of such foam materials in absorbent disposable articles has also been previously suggested.

Furthermore, it is previously known to utilize particle-shaped absorbent polymers or superabsorbents together with fluff pulp in absorbent disposable articles.

Foam materials according to prior art, which primarily absorb liquid through capillary absorption, can be perceived as having certain defects where the use in absorbent disposable articles is concerned, for example insufficient absorption capacity and liquid retaining ability.

The use of particle-shaped superabsorbents, acting through osmotic forces, in absorbent disposable articles certainly provides high absorption capacity and retention ability, but might cause problems with gel blocking and uneven liquid distribution.

SUMMARY OF THE INVENTION:

Accordingly, the object of the present invention is to provide a manufacturing method for an absorbent material structure, which combines the advantages of capillary absorption with the advantages which are achieved by means of absorption acting through osmotic forces.

In accordance with the following claim 1, the object of the invention is achieved by means of the method comprising: emulsifying an oil phase and a water phase using an emulsifier in order to form a stable emulsion, wherein the volume of the oil phase is smaller than the volume of the water phase, and the oil phase comprises at least a first polymerizable monomer having glass-like properties and/or at least a second polymerizable polymer having rubber-like properties, and a cross-linking agent, while the water phase comprises initiator and electrolyte and/or one or several polymeric hydrophilizing agents; and polymerizing the polymerizable monomer or monomers in the essentially continuous oil phase of the stable emulsion in order to form a polymeric foam structure which is elastically workable and comprises a plurality of cells with a plurality of internal surfaces, and that thereby the polymeric foam structure is hydrolized in order to create ionic groups on the internal surfaces of the polymeric foam structure in order to thereby provide an absorbent material structure with an ability for capillary absorption and absorption by means of osmotic forces. An advantageous use for the material structure produced according to the invention is for absorption of liquids which preferably are body fluids in an absorbent disposable article which comprises an absorbent material structure produced according to the invention in its absorbent body.

The absorbent material structure produced according to the invention can be manufactured using the basic principles for preparation of so-called HIPE-emulsions with subsequent polymerization of the oil phase, as has been described herein in connection with the prior art.

However, the absorbent material structure produced according to the invention differs from the above-described prior art in that the material structure according to the invention is provided with ionic groups by means of hydrolysis, something which makes it possible for the material structure according to the invention to absorb liquid both through capillary absorption and by means of absorption mechanisms acting through osmotic forces.

When manufacturing material structures according to the invention, in a similar way as described in connection with the prior art, monomers, cross-linking agents, water, and possible other additives are mixed with each other in the presence of an emulsifying agent, in order to form a HIPE-emulsion with an oil phase and a water phase.

The emulsifying agent is constituted of one or several surfactants. Examples of suitable surfactants are sorbitanmonooleate and sorbitantrioleate which, inter alia, are provided under the trademarks SPAN 80 and SPAN 85. Many other surfactants, however, are conceivable when practising the invention.

When manufacturing material structures according to the invention, acrylo-nitrile is advantageously used in the oil phase as a "glass-like" monomer raw material, but also other suitable monomers can be used, e.g. styrene. To the glass-like monomer is also advantageously added a monomer which is able to provide "rubber-like" properties to the finished material structure. This monomer is preferably 2-ethyl-hexylacrylate, but many other monomers are conceivable.

It is also possible to manufacture material structures according to the invention solely with "rubber-like" monomer raw material, for example 2-ethylhexylacrylate.

In this context, "glass-like" refers to the fact that several such monomers linked together in a polymeric chain would result in a so-called "hard segment", whereas "rubber like" refers to the fact that several such monomers linked together would result in a soft segment.

Analogous to this, several adjacent polymer chains of each of the two different types would result in a material structure with hard or glass-like regions, and in a material structure with soft or rubber-like regions.

By means of the selection of glass-like and rubber-like monomer and their mutual mixing proportion, the softness, tenacity and flexibility of the finished material structure according to the invention can be controlled.

The monomers which are utilized when manufacturing a material structure according to the invention are preferably mono-functional and contain a vinyl-bond, i.e. a C=C bond. During polymerization, the monomers form polymer chains by means of one C=C bond of a monomer being transformed into a C-C bond, which in turn reacts with a C=C bond of another monomer, etc.

When manufacturing the material structure according to the invention, a cross-linking agent which advantageously is constituted of a chemical compound with one or several difunctional groups, is also added to the oil phase. This implies that the compound contains at least two C=C bonds. Examples of cross-linking agents which can be utilized when practising the invention are divinyl benzene and ethyleneglycol dimetachrylate, but also other previously known cross-linking agents may be used, for example cross-linking agents with higher functionality than two. The task of the cross-linking agent is to cross-link different polymer chains in order to form a netted polymer structure.

During manufacture of foam materials according to the invention, the water phase comprises an initiator, for example potassium persulphate ($K_2S_2O_8$) or azoisobutyronitrile (AIBN). The task of the initiator, at a raised temperature or when it is subjected to UV-radiation, is to initiate polymerization of the components in the oil phase.

Furthermore, the water phase advantageously comprises a salt, which may be of any conventional, previously known type, as described in connection with the prior art.

When preparing an emulsion, in connection with practising the invention, the water phase and the oil phase are emulsified in the presence of the above-mentioned emulsifying agent, wherein the ratio water : oil always is greater than 1.

After emulsifying, a stable emulsion is formed which is heated in a heating oven to approx. 60 °C, wherein the polymerization of the components being part of the oil phase is initiated. After approximately 12 hours, a polymeric, porous foam structure has been formed.

According to the invention, the polymerized foam structure is thereafter hydrolized with the aid of NaOH or another suitable base. In the hydrolysis, proportions of the acrylo-nitrile groups of the foam structure are transformed into carboxylic acid groups, acrylo-amide groups and carboxylate ion-groups with sodium as a counter-ion ($COO^-Na^+$). All these groups are more hydrophilic than the original acrylo-nitrile groups.

Furthermore, the carboxylate-ion groups ($COO^-$) provide ionic groups along the polymer chains, which offer possibilities for absorption acting through osmotic forces.

Accordingly, the glass-like or hard regions of the foam structure have been rendered hydrophilic and have obtained ionic groups.

During the hydrolysis treatment, also part of the side groups in regions which originate from, for example, 2-etylhexylacrylate can be transformed into carboxylic acid groups (COOH) or carboxylate groups ($COO^-$) by means of hydrolysis of esters in the soft or rubber-like regions.

As a whole, these reaction mechanisms lend properties to the material structure according to the invention which make it possible to obtain both capillary absorption and absorption through osmotic forces.

The hydrolysis treatment according to the invention also results in a change of the glass transition temperatures $T_g$ of the polymers in the foam structure. Thereby, the hydrolysis is believed to result in an increase of the $T_g$ in the glass-like or hard regions, since polyacrylic acid, polyacrylate, and polyacryloamide each have an equal or higher $T_g$ than polyacrylonitrile. In the rubber-like or soft regions, $T_g$ has certainly been raised after the hydrolysis, since $T_g$ for polyacrylic acid and polyacrylate are above room temperature, while $T_g$ for poly(2-ethylhexylacrylate is at approximately 70 °C.

The transformation of the above-mentioned chemical groups, caused by the hydrolysis, gives rise to an increased softness and flexibility of the finished material structure according to the invention. The hydrolysis also causes the cell walls of the material structure to become thinner, resulting in an increase in the available pore volume for absorption in the material structure.

If desirable or necessary, the foam material is washed before and/or after the hydrolysis, using previously known technique suitable for the purpose, which has been described above.

Even if the hydrolysis treatment according to the invention, as mentioned above, gives rise to an increase of the number of hydrophilic groups in the material structure, the material structure according to the invention will in principle, like the previously known foam materials, have a material structure with relatively hydrophobic surfaces.

As mentioned above in connection with the description of the prior art, the hydrophobic character can be eliminated by means of different hydrophilizing treatments. According to prior art, such hydrophilizing treatment can be done by addition of surfactants or other chemical additives, both before and after polymerization of the oil phase of a HIPE-emulsion.

In order to obtain the largest possible hydrophilizing effect, and in order to avoid degradation of the hydrophilizing agent during the hydrolysis, the surfaces of the highly absorbent material structure according to the invention preferably are rendered hydrophilic after the above-mentioned hydrolysis treatment.

However, hydrophilizing treatment can also occur by means of adding, for example, hydroxy-ethylcellulose, polyacrylate or polyacrylic acid to the water phase before polymerization. Also other hydrophilizing treatments, which have been described above in connection with the prior art, can be utilized for rendering material structures according to the invention hydrophilic.

After the hydrolysis treatment and possible hydrophilizing treatment, the polymerized foam material is dried with previously known technique suitable for the purpose, whereafter a highly absorbent material structure according to the invention is obtained.

Because of the fact that the material structure according to the invention has been subjected to the above-described hydrolysis, a highly absorbent material structure in the form of a flexible, open-celled foam material is obtained. In addition to conventional capillary absorption, the material structure according to the invention also provides absorption by means of osmotic absorption mechanisms. This is made possible due to the ionic groups created by means of the hydrolysis treatment. The reduction of the thickness of the cell walls caused by the hydrolysis increases the pore volume available for absorption.

Therefore, the material structure according to the invention will obtain both an increased absorption capacity, and absorption by means of osmotic forces due to the above-mentioned inserted ionic groups. It is a known fact, that absorption acting through osmotic forces in general provides a higher liquid-retaining ability (retention ability) than capillary absorption. As has become evident above in connection with the previously known art, particle-shaped superabsorbents primarily act through osmotic forces and therefore normally offer high retention ability.

In contrast to in conventional particle-shaped superabsorbents, the ionic groups of the material structure according to the invention are bonded in the material structure, with the result that swelling and total absorption to some extent are limited in comparison with conventional superabsorbents. On the other hand, this offers the advantage that the risk of gel blocking and obstructed or uneven liquid distribution is eliminated by the material structure according to the invention.

Accordingly, in comparison with foam materials according to prior art, the absorbent material structure according to the invention provides high absorption capacity and retention ability when absorbing and distributing aqueous liquids, for example body fluids.

Therefore, the highly absorbent material structure according to the invention is perfectly well suited for applications where high retention ability and storage capacity are necessary. Examples of such advantageous applications are use in absorbent bodies for baby diapers, incontinence protectors and products for feminine hygiene.

However, the high absorption capacity and retention ability of the material structure according to the invention can also be utilized in other absorbent articles, such as for example in different wiping materials, bandage materials and other similar products.

Accordingly, absorbent material structures according to the invention have advantageous properties for use in absorbent disposable articles. Such absorbent disposable articles can be manufactured using previously known technique for manufacturing this type of products, but with the difference that material structures according to the invention, entirely or partially, replace the conventional absorbent bodies or different absorbent layers which previously have been used.

Accordingly, the material structure according to the invention also offers possibilities to manufacture absorbent disposable articles in a more rational and economically advantageous way than what previously has been possible.

In addition to highly absorbent material structures according to the invention, absorbent disposable articles according to the invention can also comprise all the other subcomponents which are present in previously known products, for example a liquid-permeable surface material on the side which is intended to be facing towards a wearer, and a liquid-impervious surface material on the side which is intended to be facing away from the wearer.

Furthermore, absorbent disposable articles according to the invention advantageously comprise elastic means, for example in the form of waist bands and elastic leg cuffs. Moreover, when desirable, absorbent disposable articles according to the invention can comprise attachment arrangements in order to facilitate dressing onto a wearer, undressing or adjustment of the size of the absorbent disposable article to the size of the wearer. Examples of such attachment arrangements are different systems with hook and loop bands, tape, hooks, eyelets or the like.

As mentioned above, hydrophilic material structures according to the invention are also suitable for use in other types of articles than diapers and sanitary napkins, for example in wiping cloths for cleaning and the like.

By means of pressing, vacuum packing or other compacting methods, highly absorbent material structures according to the invention, or absorbent disposable articles in which these are a part, can be compressed in order to facilitate storage and transportation. When material structures according to the invention are utilized in, for example, absorbent disposable articles for absorption and storage of body fluids, the articles are preferably delivered in a compressed state. When such a disposable article has been removed from its transport package and has been brought into contact with the skin of a wearer, the cells of the material structure of the invention which is part of the disposable article will, because of the elastic nature of the structure, once more have expanded to their essentially open position from having been essentially compressed during the transport.

BRIEF DESCRIPTION OF THE DRAWINGS:

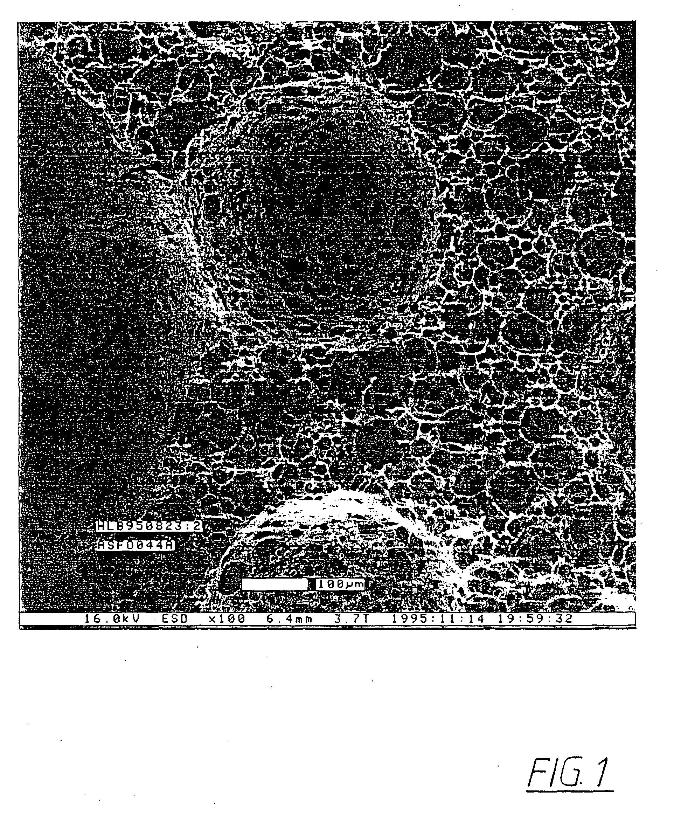

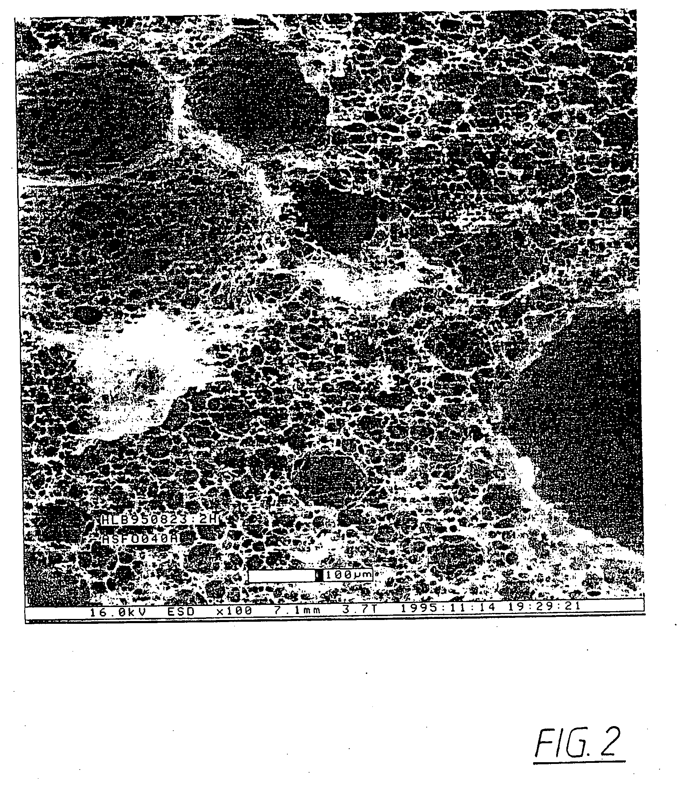

The attached figures 1 and 2 illustrate some of the differences in material structure which can be achieved by means of the invention, wherein

Fig. 1     shows a portion of a polymeric foam material in magnification which has been prepared according to prior art, and

Fig. 2     shows in magnification a portion of an absorbent material structure produced according to the invention which has been obtained by means of hydrophilizing the foam material of which a portion is shown in Fig. 1.

EXAMPLES AND PREFERRED EMBODIMENTS:

In the following, with the purpose of illustrating the invention, a number of laboratory trials will be described.

In the laboratory trials, foam materials were prepared, using the basic principles for preparation of HIPE-emulsions which have been described in the foregoing.

Thereby, when preparing hydrophilic material structures according to the invention, the working procedure was in principle as follows:

1) emulsifying,
2) polymerization,
3) washing,
4) hydrolysis,
5) washing + pH-adjustment

6) drying.

Half of the foam material obtained from each trial series was excluded from hydrolizing treatment with subsequent washing/pH-adjustment before drying, i.e. steps 4 and 5 were omitted. Thereby, the non-hydrolized foam materials can be said to constitute foam material according to prior art, while the corresponding foam materials in hydrolized condition constitute absorbent material structures according to the invention.

The emulsifying started from a standard water phase as follows:

100 g $CaCl_2$ (in the form of 137.75 g $CaCl_2*2 H_2O$)
1.5 g $K_2S_2O_8$
dist. water to 1000 ml

and a standard oil phase as follows:

0.927 g acrylonitrile
6 ml 2-ethylhexylacrylate
2 ml divinylbenzene
2 g surfactant (emulsifying agent)

The emulsifying agent in the standard oil phase was constituted of a surfactant mixture, comprising 1.8 g sorbitanmonooleate (SPAN 80) + 0.2 g sorbitantrioleate (SPAN 85).

In the laboratory trials, 300 ml of the standard water phase was poured into a polyethylene bottle and heated to 62 °C in a water bath.

Thereafter, acrylonitrile, divinylbenzene and acrylate were mixed in an emulsifying vessel of polyethylene. After addition of the surfactant (emulsifying agent), the emulsifying vessel was heated for 5 minutes in the water bath, at the temperature of 62 °C, in order to form a standard oil phase.

After this, the addition of standard water phase to the standard oil phase in the emulsifying vessel was started, via an addition funnel arranged on the emulsifying vessel. Thereby, the emulsifying vessel was placed under a $N_2$-atmosphere. An agitator, arranged for the purpose, in the emulsifying vessel was started with the speed 200 rpm. Thereafter, 10 ml of standard water phase was added to the emulsifying vessel in a single dose, whereafter 90 ml standard water phase was added drop by drop. After this, the remaining 100 ml of standard water phase was added, via the addition funnel, at a faster pace than before.

After a few minutes of additional agitation, a stable HIPE-emulsion was obtained. The emulsion was poured into a polymerization mould intended for the purpose, consisting of a rectangular box of polyethylene, in which the emulsion was allowed to polymerize during 18 h at 62 °C.

After finished polymerization, the foam material was washed with 1% $CaCl_2$. After the washing, residual liquid was pressed out from the foam material using absorbent paper.

After washing, half of the sample was dried at 62 °C during 4 h in order to provide a foam material according to prior art, for use as a reference.

In trials which were aimed at preparing absorbent material structures according to the invention, the polymerized foam material was subjected to a hydrolysis stage after washing, wherein the second half of the polymerized foam material was hydrolized with 17.5 ml 0.5 M NaOH during 16 h at 95 °C.

Thereafter, the hydrolized foam material was washed in a combined washing and pH-adjustment stage to pH 7.

Finally, the hydrolized and pH-adjusted foam material was dried at 62 °C during 4 h, in order to provide an absorbent material structure according to the invention.

With the above-described basic recipe and basic method as a starting point, a trial series was performed by means of which the influence of different parameters on the processability and the finished material structure was evaluated. Thereby, the following parameters were chosen for evaluation; cross-linking agents, emulsifying agents, agitation and design of agitators, HLB-value ("Hydrophilic/Lipophilic Balance"), other additives, monomers and hydrolysis conditions.

In all trial series, the influence of the different parameters on a foam material according to prior art, and on absorbent material structures according to the invention were compared, by means of exempting half of each polymerized material batch from hydrolysis (prior art) and subjecting the other half of each polymerized material batch to hydrolysis (according to the invention).

The finished foam materials were evaluated using a number of testing methods. Amongst the testing methods from which results will be reported herein, the following can be mentioned:

Density, was determined by means of a gravimetric procedure in which the weight and the volume of a foam material specimen is determined, after which the density is calculated and expressed in the unit g/cm$^3$.

<u>Wet density</u>, was determined in a corresponding way to density, but starting from wet weight and wet volume after absorption of synthetic urine.

<u>Absorption capacity</u>, was determined by means of weighing a foam material specimen in dry state, and by thereafter immersing the material specimen during 1 h into a liquid container with synthetic urine of a conventional type. After the material sample had been removed from the liquid container, excess liquid was allowed to run off, whereafter the wet material specimen was weighed. Finally, the absorption capacity was calculated according to; absorbed liquid (g) / dry weight of material specimen (g), and reported in the unit g/g.

The synthetic urine which was utilized when testing foam materials was of a conventional type, comprising different salts intended to imitate the chemical composition of human urine. In principle, similar test results could be reached using any of the commercially available types of synthetic urine, which are well known to the skilled person.

Some of the results from the different trial series are evident from the following Table 1. In the table, trials based on the above-mentioned standard emulsion are reported, as well as trials based on emulsions with only "rubber-like" 2-ethylhexylacrylate as monomer.

It is evident from the results in Table 1 that foam materials which have been subjected to hydrolysis, i.e. material structures according to the invention, offer lower dry and wet density, and clearly higher absorption capacity than corresponding, non-hydrolized foam materials according to prior art.

Part of the detected increased absorption capacity of the material structures according to the invention originates from an increased available pore volume for absorption, primarily caused by the reduction of the wall thickness of the cells in the material structure created by the hydrolizing treatment.

It has also been found that the hydrolizing treatment according to the invention is able to create new pores in portions which before hydrolysis are constituted of compact, polymeric foam material. This gives a further contribution to the increased absorption capacity of material structures according to the invention. This effect can be discerned in a comparison between the two attached Figs. 1 and 2.

Furthermore, part of the increase of the measured absorption capacity of the material structures according to the invention can be derived from increased retention ability due to absorption through osmotic forces caused by the above-mentioned ionic groups created in the hydrolysis.

As mentioned above, with the purpose of illustrating the invention, a trial series with different HLB-values (HLB = Hydrophilic/Lipophilic Balance) was also performed. Thereby, the HLB-value can be said to be a measure of the balance between hydrophilic and hydrophobic character of the emulsifying agent used in the respective trials. The HLB-value for mixtures of different surfactants which were used as emulsifying agents was calculated according to:

$$HLB_{tot} = m_1 \; / \; m_{tot} \; * \; (HLB_1 \; - \; HLB_2) \; + \; HLB_2$$

wherein
$HLB_{tot}$: is the total HLB-value of the surfactant mixture,
$m_1$: is the added quantity (i.e. weight) in g of surfactant 1,
$m_{tot}$: is the total added quantity of surfactant in g (surfactant 1 + 2)
$HLB_1$: is the HLB-value reported by the manufacturer for surfactant 1, and
$HLB_2$: is the HLB-value reported by the manufacturer for surfactant 2.

The results from the trial series, aiming at evaluating the influence of the HLB-value on the obtained foam structure, measured as pore volume available for absorption (absorption capacity), are evident from the following Table 2. In the trial series, the above-defined standard phases were used as a starting point, wherein the quantities and proportions of the surfactants SPAN 80 and SPAN 85 in the emulsifying agent were varied in order to obtain different HLB-values. Thereby, SPAN 80 had a HLB-value reported by the manufacturer of 4.3, while the corresponding value for SPAN 85 was 1.8. The calculation of the total HLB-value of the emulsifying agent was performed in the way described above.

As is evident from Table 2, hydrolizing treatment according to the invention provided material structures with lower wet density and clearly higher absorption capacity than corresponding non-hydrolized foam materials according to prior art.

Furthermore, it is evident that the $HLB_{tot}$-value is of great importance for the properties which are to be obtained in the finished foam materials, both with regard to material structures which have been hydrolized according to the invention and to non-hydrolized foam materials according to prior art.

Results from the trials accounted for herein, and from further trials, have shown that the most advantageous $HLB_{tot}$-values for the application in question are within the interval 3.8 to 4.3. With an $HLB_{tot}$-value in this region, the highest absorption capacity for material structures according to the invention will be obtained.

Whee the other factors which were studied during the different laboratory trials are concerned, it can be mentioned that the total amount of surfactant in the emulsifying agent affects the properties of the prepared material structures. With regard to the surfactants which primarily were used in the herein described trials, SPAN 80 and 85, the most advantageous results were obtained when the total surfactant amount was between 1.5 and 2.5 g in the above-mentioned standard oil phase. As should be evident to the person skilled in the art, the surfactant addition has to be adjusted to the specific conditions which prevail in each individual case when the invention is practised.

The type of cross-linking agent which is used and the dosage also proved to have an effect on the obtained material structures. Thereby, it can be mentioned that divinylbenzene gave the most advantageous results for the application in question.

Furthermore, the agitating speed, within the interval 150 350 rpm, proved to have a proportionately small effect, whereas the design of the agitator had a relatively large effect on the properties of the obtained material structures according to the invention.

The best results, for the application in question, were obtained with flat agitators having cut-out portions. The trials proved that when emulsifying it is essential that the dimensions of the agitator are adapted to the dimensions of the emulsifying vessel, so that good turbulence is obtained in the entire emulsifying volume. Propeller agitators gave worse results with regard to the absorption capacity of the finished material structures according to the invention.

The influence of the emulsifying temperature on the material structure was evident in such a way that temperatures around room temperature provided proportionately compact structures with high density and low absorption capacity, while temperatures around or higher than 60 °C provided material structures with lower density and higher absorption capacity. Accordingly, higher temperatures provided material structures with more advantageous properties for the application in question. When preparing material structures according to the invention, the emulsifying temperature is advantageously between 25 and 65 °C, and preferably between 60 and 65 °C.

With a view to increasing the hydrophilic character of the finished material structure, a number of trials with additions of hydroxy-ethyl cellulose, polyacrylic acid and polyacrylate to the emulsifying mixture were performed.

The results from these trials did not result in any greater differences in hydrophilic character or absorption capacity, but the effect of the subsequent hydrolizing treatment according to the invention clearly overshadowed the effect provided by other chemical additives.

Trials with different monomers proved that the invention can be successfully practised with several different monomers or mixtures of monomers as a base. Examples of suitable monomer raw materials for preparation of highly absorbent material structures according to the invention are acrylonitrile, ethylhexyl-acrylate, acrylonitrile + ethylhexyl-acrylate, styrene, styrene + ethylhexylacrylate, but also other monomers are conceivable.

Trials with different hydrolizing methods proved that good material properties can be obtained with a hydrolizing method according to what has been described in the foregoing, using 0.5 M NaOH, but also other strong bases can be utilized for the hydrolizing treatment.

It has to be understood that the volumes of different raw materials and other trial conditions reported in the embodiments should be seen as possible examples of what makes it possible to practice the invention, and that an industrial manufacture of highly absorbent material structures according to the invention requires an adaptation to the prevailing conditions. Such an adaptation, however, is fully accomplishable for a person skilled in the art.

The present invention should not be regarded as being limited to what has been disclosed in the form of examples, tables or figures, but its scope is defined by the attached claims.

TABLE 1

| Trial | Hydrolizing treatment acc. to the invention | No. | Density ($g/cm^3$) | Wet density ($g/cm^3$) | Absorption capacity ($g/g$) |
|---|---|---|---|---|---|
| standard oil phase + standard water phase | NO | 950823:1 | 0.500 | 1.035 | 8.33 |
| | YES | 950823:1H | 0.043 | 0.907 | 24.84 |
| | NO | 950823:2 | 0.112 | 0.907 | 8.63 |
| | YES | 950823:2H | 0.035 | 0.884 | 27.24 |

(continued)

| 7.12 g 2-ethylhexyl acrylate 1.82 g divinylbenzene 1.5 g SPAN 80 0.5 g SPAN 85 + standard water phase | NO | 950612:1 | 0.076 | 0.547 | 6.24 |
| | YES | 950612:1H | 0.039 | 0.461 | 11.25 |
| | NO | 950627 | 0.125 | 0.840 | 6.06 |
| | YES | 950627H | 0.055 | 0.673 | 13.29 |

TABLE 2

| Trial | Hydrolizing treatment acc. to the invention | No. | $HLB_{tot}$ | Wet density (g/cm$^3$) | Absorption capacity (g/g) |
|---|---|---|---|---|---|
| standard oil phase + standard water phase - $HLB_{tot}$ was varied by means of varying the quantities and proportions of the surfactants 80 and SPAN 85 being part of the emulsifying agent | NO | 950908:1 | 4.3 | 0.995 | 6.18 |
| | YES | 950818:1H | 4.3 | 0.956 | 21.32 |
| | NO | 950823:1 | 4.05 | 1.035 | 8.33 |
| | YES | 950823:1H | 4.05 | 0.907 | 24.84 |
| | NO | 950823:2 | 4.05 | 0.907 | 8.63 |
| | YES | 950823:2H | 4.05 | 0.884 | 27.24 |
| | NO | 950824:2 | 3.92 | 0.998 | 9.63 |
| | YES | 950824:4H | 3.8 | 1.042 | 18.02 |
| | YES | 950911:2H | 8.6 | 0.239 | 1.14 |
| | NO | 950911:3 | 4.7 | 1.116 | 0.35 |
| | YES | 950911:3H | 4.7 | 0.363 | 0.42 |

**Claims**

1. A method of manufacturing an absorbent material structure for absorbing liquids, comprising: emulsifying an oil phase and a water phase using an emulsifier in order to form a stable emulsion, wherein the volume of said oil phase is smaller than the volume of said water phase, and said oil phase comprises at least a first polymerizable monomer having glass-like properties and/or at least a second polymerizable polymer having rubber-like properties and a cross-linking agent, while said water phase comprises initiator and electrolyte; and polymerizing said polymerizable monomer or monomers in the essentially continuous oil phase of the stable emulsion in order to form a polymeric foam structure which is elastically workable and comprises a plurality of cells with a plurality of internal surfaces,
**characterized in that** the polymeric foam structure is hydrolized by means of basic hydrolysis in order to create ionic groups on the internal surfaces of the polymeric foam structure in order to thereby provide an absorbent material structure with an ability for capillary absorption and absorption through osmotic forces.

2. A method according to claim 1,
**characterized in that** the water phase comprises one or several polymeric hydrophilizing agents.

3. A method according to claim 1 or 2,
**characterized in that** emulsifying takes place with the use of a plate-shaped agitator with cut-out portions and that the dimensions of the agitator correspond to the dimensions of an emulsifying vessel so that turbulence arises in essentially the entire liquid volume of said emulsifying vessel.

4. A method according to claim 1, 2 or 3,
**characterized in that** emulsifying takes place at a temperature within the range of 25 to 65 °C, and preferably within the range of 60 to 65 °C.

**5.** A method according to any one of claims 1 to 4,
**characterized in that** the absorbent material structure is subjected to washing and/or pH-adjustment after finished hydrolysis.

**Patentansprüche**

**1.** Verfahren zur Herstellung einer absorbierenden Materialstruktur zum Absorbieren von Flüssigkeiten, umfassend: Emulgieren einer Ölphase und einer Wasserphase unter Verwendung eines Emulgators, um eine stabile Emulsion zu bilden, wobei das Volumen der Ölphase kleiner als das Volumen der Wasserphase ist und die Ölphase zumindest ein erstes polymerisierbares Monomer mit glasigen bzw. glasähnlichen Eigenschaften und/oder zumindest ein zweites polymerisierbares Polymer mit gummiartigen Eigenschaften und ein Vernetzungsmittel umfaßt, wobei die Wasserphase Initiator und Elektrolyt umfaßt; und Polymerisieren des polymerisierbaren Monomers oder der Monomere in der im wesentlichen kontinuierlichen Ölphase der stabilen Emulsion, um eine polymere Schaumstruktur zu bilden, die elastisch ausführbar ist und eine Vielzahl von Zellen mit einer Vielzahl von inneren Oberflächen umfaßt, **dadurch gekennzeichnet, daß** die polymere Schaumstruktur mittels basischer Hydrolyse hydrolysiert wird, um ionische Gruppen auf den inneren Oberflächen der polymeren Schaumstruktur zu bilden, um hierdurch eine absorbierende Materialstruktur mit einer Fähigkeit zur kapillaren Absorption und Absorption durch osmotische Kräfte bereitzustellen.

**2.** Verfahren gemäß Anspruch 1, **dadurch gekennzeichnet, daß** die Wasserphase ein oder mehrere polymere(s) Hydrophilisierungsmittel umfaßt.

**3.** Verfahren gemäß Anspruch 1 oder 2, **dadurch gekennzeichnet, daß** das Emulgieren unter Verwendung eines plattenförmigen Rührers mit herausgeschnittenen Teilen durchgeführt wird und daß die Dimensionen des Rührers den Dimensionen eines Emulgiergefäßes entsprechen, so daß sich Turbulenzen im wesentlichen in dem gesamten Flüssigkeitsvolumen des Emulgiergefäßes ergeben.

**4.** Verfahren gemäß Anspruch 1, 2 oder 3, **dadurch gekennzeichnet, daß** das Emulgieren bei einer Temperatur im Bereich von 25 bis 65°C, und bevorzugt im Bereich von 60 bis 65°C, stattfindet.

**5.** Verfahren gemäß irgendeinem der Ansprüche 1 bis 4, **dadurch gekennzeichnet, daß** nach der vollendeten Hydrolyse die absorbierende Materialstruktur einem Waschen und/oder einer pH-Einstellung unterzogen wird.

**Revendications**

**1.** Procédé de fabrication d'une structure de matière absorbante pour absorber des liquides, comprenant : l'émulsification d'une phase huileuse et d'une phase aqueuse utilisant un émulsifiant afin de former une émulsion stable, où le volume de ladite phase huileuse est inférieur au volume de ladite phase aqueuse, et ladite phase huileuse comprend au moins un premier monomère polymérisable ayant des propriétés similaires au verre et/ou au moins un deuxième polymère polymérisable ayant des propriétés similaires au caoutchouc et un agent de réticulation, alors que ladite phase aqueuse comprend un initiateur et un électrolyte ; et la polymérisation dudit monomère ou desdits monomères polymérisables dans la phase huileuse sensiblement continue de l'émulsion stable afin de former une structure de mousse polymère qui est façonnable élastiquement et comprend une pluralité de cellules avec une pluralité de surfaces internes, **caractérisé en ce que** la structure de mousse polymère est hydrolysée au moyen d'une hydrolyse basique afin de créer des groupes ioniques sur les surfaces internes de la structure de mousse polymère de manière à fournir ainsi une structure de matière absorbante avec une capacité d'absorption capillaire et d'absorption grâce aux forces osmotiques.

**2.** Procédé selon la revendication 1, **caractérisé en ce que** la phase aqueuse comprend un ou plusieurs agents d'hydrophilisation polymériques.

**3.** Procédé selon la revendication 1 ou 2, **caractérisé en ce que** l'émulsification a lieu avec l'utilisation d'un d'agitateur de forme plane avec des parties découpées et **en ce que** les dimensions de l'agitateur correspondent aux dimensions d'un récipient d'émulsification de sorte qu'une turbulence apparaisse essentiellement dans la totalité du volume de liquide dudit récipient d'émulsification.

**4.** Procédé selon la revendication 1, 2 ou 3, **caractérisé en ce que** l'émulsification a lieu à une température comprise dans le domaine de 25°C à 65°C, et de préférence dans le domaine de 60°C à 65°C.

**5.** Procédé selon l'une quelconque des revendications 1 à 4, **caractérisé en ce que** la structure de matière absorbante est soumise à un lavage et/ou à un ajustement de pH une fois l'hydrolyse terminée.

FIG. 1

*FIG. 2*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9321234 A
- WO 9304092 A
- WO 9304093 A
- WO 9304113 A
- WO 9304115 A
- WO 9413704 A
- US 5500451 A
- EP 0156541 A